# EUROPÄISCHE PATENTSCHRIFT

(11) **EP 0 502 376 B1**
(45) Veröffentlichungstag und Bekanntmachung des Hinweises auf die Patenterteilung: **30.11.1994**
(21) Anmeldenummer: 92102915.3
(22) Anmeldetag: 21.02.1992
(51) Int. Cl.: C07C 45/60, C07C 45/59, C07C 47/232, C07C 47/277, C07C 403/14

(54) **Verfahren zur Herstellen alpha, beta-ungesättigter Aldehyde und Ketone**
Method for the production of alpha,beta-unsaturated aldehydes and ketones
Procédé pour la fabrication d'aldéhydes et de cétones alpha,bêta-insaturées

(30) Priorität: 05.03.1991 DE 4106908
(43) Veröffentlichungstag der Anmeldung: 09.09.1992
(73) Patentinhaber: BASF Aktiengesellschaft, 67063 Ludwigshafen (DE)
(72) Erfinder: Mackenroth, Christiane, W-6702 Bad Duerkheim (DE); Buschmann, Ernst, Dr., W-6700 Ludwigshafen (DE)

(56) Entgegenhaltungen:
- SYNTHESIS. Juli 1981, STUTTGART DE Seiten 501 - 522; F.A.J. MESKENS: 'Methodsfor the preparation of acetals from alcohols or oxiranes and carbonylcompounds'
- W. THEILHEIMER 'Synthetische Methoden der organischen Chemie Band 4' 1966 ,S. KARGER , BASEL, CH
- HOUBEN-WEYL 'Methoden der organischen Chemie Band VI/3' 1965 , GEORG THIEMEVERLAG , STUTTGART DE
- T. W. GREENE 'Protective groups in organic synthesis' 1981 , JOHN WILEY& SONS INTERSCIENCE , NEW YORK, USA

## Beschreibung

Die vorliegende Erfindung betrifft ein neues Verfahren zur Herstellung von α,β-ungesättigten Aldehyden und Ketonen der allgemeinen Formel I,

O=C(R¹)-CR²=CR³R⁴ I

in der die Substituenten R¹, R², R³ und R⁴ unabhängig voneinander für Wasserstoff, ggf. subst. Alkyl- oder ggf. subst. Arylgruppen stehen, durch saure Hydrolyse von entsprechenden cyclischen α,β-ungesättigten Acetalen der allgemeinen Formel II, 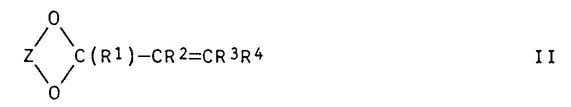
in der Z für eine ggf. subst. C₂- oder C₃-Kohlenstoffkette steht.

Aus der Literatur ist bekannt, daß man Acetale durch saure Hydrolyse in die entsprechenden Aldehyde überführen kann (Houben-Weyl, Methoden der organischen Chemie, Bd. XII/1, Seite 434ff). Diese Umsetzung verläuft nicht vollständig und muß daher mehrmals wiederholt werden. Im Fall von thermisch labilen Verbindungen, z.B. α,β-ungesättigten Verbindungen treten dadurch erhebliche Verluste an Wertprodukt auf.

Des weiteren ist ein Verfahren zur Herstellung β,γ-ungesättigter Aldehyde bekannt, das darauf beruht, daß man ein entsprechendes cyclisches Acetal zunächst mit Methanol in saurem Medium in das Dimethylacetal überführt, welches anschließend vollständig in bekannter Weise hydrolysiert werden kann (Stowell, Synthesis 1979, Seite 132ff) . Ausgehend von α,β-ungesättigten Acetalen erhält man nach diesem Verfahren komplexe Produktgemische.

Nach einer weiteren literaturbekannten Methode erhält man Aldehyde und Ketone aus den entsprechenden Acetalen durch saure Hydrolyse in Gegenwart von Aceton (Houben-Weyl, Methoden der organischen Chemie, Bd. VI/3, Seite 278ff) . Dieses Verfahren angewendet auf α,β-ungesättigten Acetale liefert keinen vollständigen Umsatz.

Es ist weiter bekannt, daß Ketone aus den entsprechenden Acetalen durch saure Hydrolyse in Gegenwart von Aldehyden oder Ketonen erhalten werden (Meskens, Synthesis 1981, Seite 501ff) und daß aus Propanal und Aceton-dimethylacetal unter Mitwirkung von p-Toluolsulfonsäure Propanal-dimethylacetal erhalten wird (Houben-Weyl, Methoden der organischen Chemie, Bd. VI/3, Seiten 253-254).

Aufgabe der vorliegenden Erfindung war ein einfaches und wirtschaftlich anwendbares Verfahren zur Herstellung α,β-ungesättigter Aldehyde und Ketone.

Demgemäß wurde ein Verfahren zur Herstellung α,β-ungesättigter Aldehyde und Ketone der allgemeinen Formel I,

O=C(R¹)-CR²=CR³R⁴ I

in der die Substituenten R¹, R², R³ und R⁴ unabhängig voneinander für Wasserstoff, ggf. subst. Alkyl- oder ggf. subst. Arylgruppen stehen, durch saure Hydrolyse von entsprechenden cyclischen α,β-ungesättigten Acetalen der allgemeinen Formel II, 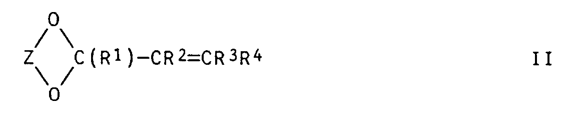
in der Z für eine ggf. subst. C₂- oder C₃-Kohlenstoffkette steht, gefunden, welches dadurch gekennzeichnet ist, daß man die Hydrolyse in Gegenwart von gesättigten Aldehyden durchführt.

Im Vergleich mit den literaturbekannten Verfahren liefert das neue Verfahren erheblich bessere Ausbeuten an Produkt.

Als erfindungsgemäß für die Acetalspaltung zu verwendende gesättigte Aldehyde eignen sich insbesondere aliphatische Aldehyde, wobei die Kohlenstoff-Kettenlänge und der Grad der Verzweigung der Kohlenstoff-Kette sowie gegen die Reaktionsbedingungen der Umsetzung inerte Substituenten wie Alkoxygruppen und Alkylcarbonyloxygruppen nach den bisherigen Erkenntnissen keinen Einfluß auf das Verfahren haben.

Im allgemeinen wird die Art des zu verwendenden gesättigten Aldehyds von den folgenden Aspekten abhängig sein:
- Der Aldehyd sollte leicht zugänglich sein;
- der Aldehyd sollte unter den Hydrolysebedingungen nicht mit dem im Reaktionsmedium vorliegenden Wertprodukt Reaktionen eingehen können;
- der Aldehyd sollte unter den Hydrolysebedingungen nicht zu einem Produkt reagieren, welches sich vom Wertprodukt schlecht abtrennen läßt.

Aus den oben genannten Erwägungen verwendet man üblicherweise unverzweigte oder verzweigte C₁-C₆-Aldehyde wie Formaldehyd, Acetaldehyd, Propionaldehyd, Butyraldehyd, α-Methylpropionaldehyd, Pentanal, α-Methylbutyraldehyd, β-Methylbutyraldehyd, α,α-Dimethylpropionaldehyd, Hexanal, α-Methylpentanal, β-Methylpentanal, γ-Methylpentanal, α,α-Dimethylbutyraldehyd, α,β-Dimethylbutyraldehyd, β,β-Dimethylbutyraldehyd und α-Ethylbutyraldehyd, vorzugsweise unverzweigte C₃-C₅-Aldehyde, insbesondere Propionaldehyd.

Der gesättigte Aldehyd wird im allgemeinen mindestens in äquimolaren Mengen bezogen auf die Verbindung II eingesetzt, da er der Umsetzungsgleichung entsprechend in molaren Mengen verbraucht wird. Nach bisheriger Kenntnis führt die Verwendung von größeren Überschüssen an gesättigtem Aldehyd zu keinem zusätzlichen vorteilhaften Effekt für das Verfahren. Üblicherweise verwendet man 1 bis 3 mol-Äq., vorzugsweise 1 bis 2 mol-Äq., insbesondere 1 bis 1,2 mol-Äq. bezogen auf II.

Das erfindungsgemäße Verfahren wird im allgemeinen in Anlehnung an die bekannten Verfahren zu Acetalspaltung (Hydrolyse) in einem inerten aprotischen organischen Lösungsmittel in Gegenwart von Säuren durchgeführt.

Die Umsetzung wird bei Temperaturen von 0 bis 150°C, vorzugsweise 20 bis 100°C, insbesondere 50 bis 70°C durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Tetrahydrofuran.

Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, EisenIII-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung.

Die Säuren werden im allgemeinen in katalytischen Mengen eingesetzt. Vorteilhafterweise verwendet man die Säuren in Mengen von 0,1 bis 1 mol-Äq., vorzugsweise 0,2 bis 0,8 mol-Äq., insbesondere 0,4 bis 0,6 mol-Äq. bezogen auf das eingesetzte Acetal II.

Die Aufarbeitung der Reaktionsmischung und die Isolierung des Produktes erfolgt in an sich bekannter Weise, indem zunächst die Säure aus der Reaktionsmischung entfernt wird und aus der so erhaltenen Reaktionslösung anschließend durch Kristallisation, Chromatographie oder Destillation das Produkt isoliert wird.

Das erfindungsgemäße Verfahren eignet sich zur Herstellung von α,β-ungesättigten Aldehyden bzw. Ketonen I und substituierten Derivaten aus den entsprechenden alpha,beta-ungesättigten Acetalen II, insbesondere solchen der allgemeinen Formel IIa 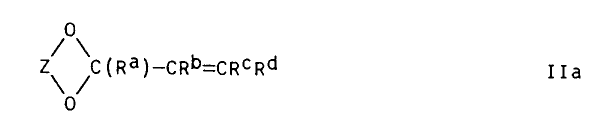
in welcher die Substituenten folgende Bedeutung haben:
R^{a}, R^{b}, R^{c} und R^{d} unabhängig voneinander
Wasserstoff oder C-organische Reste wie Alkyl-, Alkenyl-, Alkinyl- und Arylgruppen; gegen die Reaktionsbedingungen inerte Substituenten an diesen C-organischen Resten haben nach den bisherigen Erkenntnissen keinen Einfluß auf das Verfahren;
Bevorzugt kommen als Reste R^{a}, R^{b}, R^{c} und R^{d} folgende Gruppen in Betracht:
Wasserstoff;
Alkylgruppen mit bis zu zwanzig C-Atomen, insbesondere C₁-C₄-Alkylgruppen wie Methyl, Ethyl, Propyl, 1-Methylethyl, Butyl, 1-Methylpropyl und 2-Methylpropyl;
Alkenylgruppen mit bis zu zwanzig C-Atomen, insbesondere C₂-C₄-Alkenylgruppen wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl und 2-Methyl-2-propenyl;
Alkinylgruppen mit bis zu zwanzig C-Atomen, insbesondere C₂-C₄-Alkinylgruppen wie Ethinyl, 1-Propinyl, 2-Propinyl, 1-Butinyl, 2-Butinyl, 3-Butinyl und 1-Methyl-2-propinyl;
und Arylgruppen wie insbesondere Phenyl.

Die vorstehend genannten Reste können ihrerseits durch Heteroatome wie Stickstoff, Sauerstoff und Schwefel unterbrochen sein oder weitere inerte Reste wie Halogen, Nitro, Sulfonyl, Arylsulfonyl, Carboxyl, Cycloalkyl oder Cycloalkenyl tragen.

Die Gruppe Z steht für eine gegebenenfalls substitutierte C₂- oder C₃-Kohlenstoffkette, insbesondere -CH₂-C(CH₃)₂-CH₂-.

Die nach dem erfindungsgemäßen Verfahren besser zugänglichen α,β-ungesättigten Aldehyde bzw. Ketone I und deren Derivate dienen beispielsweise als Zwischenprodukte in der Herstellung von Pharmaka, Farben und Pflanzenschutzmitteln.

Im Hinblick auf ihre Verwendung als Zwischenprodukte für Pflanzenschutzmittel sind insbesondere die α,β-ungesättigten Aldehyde und Ketone Ia,

O=C(R^{a})-CR^{b}=CR^{c}R^{d} Ia

bevorzugt, in der die Substituenten folgende Bedeutung haben:
R^{a}, R^{b}, R^{c} und R^{d} unabhängig voneinander;
Wasserstoff;
C₁-C₂₀-Alkyl wie Methyl, Ethyl, Propyl, 1-Methylethyl, Butyl, 1-Methylpropyl, 2-Methylpropyl, 1, 1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1, 2-Dimethylpropyl, 1-Methylpentyl, 2-Methylpentyl, 3-Methylpentyl, 4-Methylpentyl, 1, 1-Dimethylbutyl, 1, 2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Man erhält diese Verbindungen Ia beispielsweise dadurch, daß man das Acetal einer alpha-Brom Carbonylverbindung der allgemeinen Formel IIIa in an sich bekannter Weise mit Triphenylphosphin in das entsprechende Triphenylphosphonium-Salz IVa umwandelt, das so erhaltene Salz anschließend in an sich bekannter Weise im Sinne einer Wittig-Reaktion mit einem Aldehyd oder einem Keton der allgemeinen Formel Va umsetzt und das dadurch erhaltene α,β-ungesättigten Acetal IIa erfindungsgemäß in den α,β-ungesättigten Aldehyd bzw. das Keton Ia spaltet. 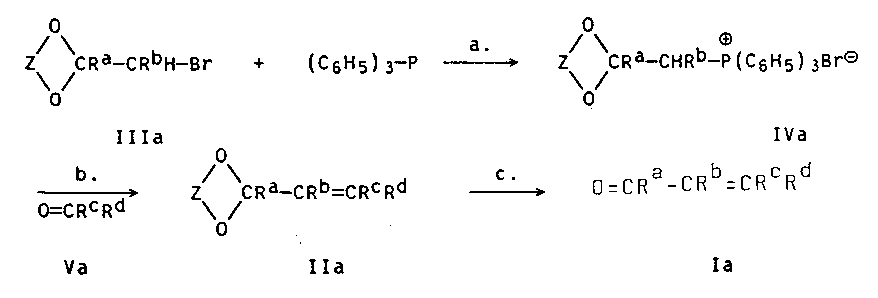
Die Umsetzungen a, b und c werden wie folgt durchgeführt:

### a) Herstellung des Phosphoniumsalzes (Sargent et al., J. Chem. Soc. PTI, 1974, 37f.)

Die Umsetzung erfolgt im allgemeinen bei Temperaturen von 100 bis 200°C, vorzugsweise von 100 bis 160°C.

Als Lösungsmittel eignen sich beispielsweise Toluol, o-, m-, p-Xyol und Dimethylformamid, insbesondere Toluol.

### b) Wittig-Reaktion (Sargent et al., J. Chem. Soc. PTI, 1974, 37f.)

Die Reaktion erfolgt im allgemeinen bei Temperaturen von 20 bis 100°C, vorzugsweise von 20 bis 30°C.

Als Lösungsmittel eignen sich beispielsweise die vorstehend genannten, insbesondere Dimethylformamid.

### c) Acetalspaltung

Die Acetalspaltung erfolgt nach dem eingangs beschriebenen erfindungsgemäßen Verfahren.

Die α,β-ungesättigten Aldehyde bzw. Ketone der allgemeinen Formel Ia können zur Synthese von Schädlingsbekämpfungsmitteln, insbesondere zu Synthese von Pheromonen verwendet werden. In diesem Sinne werden sie in an sich bekannter Weise in die Diene VI überführt, die als Sexuallockstoffe einiger Lepidoptera-Spezies bekannt sind.

Diese Synthese ist schematisch im folgenden Umsetzungsschema zusammengefaßt: 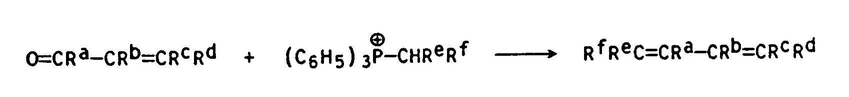
Derartige Wirkstoffe werden beispielsweise in der DE-A 38 17 399 beschrieben.

Weiterhin können α,β-ungesättigte Aldehyde bzw. Ketone der allgemeinen Formel I zur Synthese von Carotinoiden verwendet werden, in der die Substituenten folgende Bedeutung haben:
Z
Ethylen- oder Propylenrest, der durch C₁-C₄-Alkylgruppen, vorzugsweise Methylgruppen substituiert sein kann
R¹, R² und R³
H oder C₁-C₄-Alkyl
R¹
vorzugsweise H
R²
vorzugsweise H oder -CH₃
R³
vorzugsweise H oder -CH₃
R⁴
Polyenkette mit 4 bis 20 C-Atomen, die durch C₁-C₄-Alkylgruppen, vorzugsweise Methylgruppen sowie die Gruppe 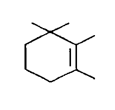
substituiert sein kann, wobei der Cyclohexenring auch Sauerstoffunktionen wie eine Oxogruppe und/oder eine Alkoxygruppe bzw. Hydroxygruppe tragen kann, vorzugsweise
- R¹ =: H
- R² =: H oder CH₃
- R³ =: H oder CH₃
- R⁴ =:

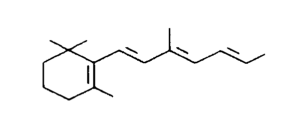

### Experimenteller Teil

Beispiele 2, 4, 6, 7 und 8 sind Vergleichsbeispiele.

### Beispiel 1

### Herstellung von Zimtaldehyd

O=CH-CH=CH-C₆H₅

Eine Lösung aus 10 g (46 mmol) Zimtaldehyd-neopentylacetal in 20 ml Tetrahydrofuran wurde bei 25°C nacheinander zunächst mit 1,67 g (46 mmol) 10 %-iger Salzsäure und anschließend mit 2,67 g (46 mmol) Propionaldehyd versetzt. Nach 20 h Rühren bei 25°C wurde die Reaktionsmischung mit Wasser versetzt. Man erhielt das Produkt durch Extraktion mit t.-Butylmethylether aus der organischen Phase.

Man erhielt so 7,2 g Rohprodukt, welches nach GC-Analyse 72,4 % Zimtaldehyd enthielt (Ausbeute 86 %).

### Beispiel 2

### Herstellung von Zimtaldehyd

O=CH-CH=CH-C₆H₅

Analog zu den in Beispiel 1 beschriebenen Bedingungen aber in Abwesenheit von Propionaldehyd erhielt man 9 g Rohprodukt, welches nach GC-Analyse 38,5 % Zimtaldehyd enthielt (Ausbeute 57 %). Außerdem enthielt dieses Rohprodukt 50,9 % an unumgesetztem Zimtaldehyd-neopentylacetal.

### Beispiel 3

### Herstellung von 3-(4-(1, 1-Dimethylethyl)-phenyl)-2-methyl-propenal

O=CH-C(CH₃)=CH-(4-C(CH₃)₃)-C₆H₄

Eine Lösung aus 28,8 g (100 mmol) 3-(4-(1, 1-Dimethylethyl)-phenyl)-2-methyl-propenal-neopentylacetal in 100 ml Tetrahydrofuran wurde bei 25°C nacheinander zunächst mit 3,65 g (10 mmol) 10 %-iger Salzsäure und anschließend mit 5,8 g (100 mmol) Propionaldehyd versetzt. Nach 3 h Rühren bei 60°C wurde die Reaktionsmischung wie bei Beispiel 1 angegeben aufgearbeitet. Man erhielt so 23 g Rohprodukt, welches nach GC-Analyse 79,8 % 3-(4-(1, 1-Dimethylethyl)-phenyl)-2-methyl-propenal enthielt (Ausbeute 91 %).

### Beispiel 4

### Herstellung von 3-(4-(1, 1-Dimethylethyl)-phenyl)-2-methyl-propenal

O=CH-C(CH₃)=CH-(4-C(CH₃)₃)-C₆H₄

Analog zu den in Beispiel 3 beschriebenen Bedingungen aber in Abwesenheit von Propionaldehyd erhielt man 19,5 g Rohprodukt, welches nach GC-Analyse 54,5 % 3-(4-(1,1-Dimethylethyl)-phenyl)-2-methyl-propenal enthielt (Ausbeute 66 %). Außerdem enthielt dieses Rohprodukt 20,0 % an unumgesetztem 3-(4-(1,1-Dimethylethyl)-phenyl)-2-methyl-propenal-neopentylacetal

### Beispiel 5

### Herstellung von 9-Acetoxynonenal und 9-Hydroxynonenal

O=CH-CH=CH-(CH₂)₅-CH₂-OR (R = H, H₃CCO)

Eine Mischung aus 144,1 g (0,55 mol) Triphenylphosphin, 118,6 g (0,55 mol) 2-Brommethyl-5,5-dimethyl-1-,3-dioxan (96,9 %-ig) 200 ml Xylol und 200 ml Dimethylformamid wurde 8 h bei 130°C gerührt. Nach dem Abkühlen auf 25°C wurde die so erhaltene Reaktionsmischung mit 67,2 g (0,6 mol) Kaliumtert.-butylat versetzt. Nach 2 h Rühren bei 25°C wurden der Mischung 90,5 g (0,5 mol) 7-Acetoxyheptanal (95 %-ig) zugemischt und die so erhaltene Reaktionsmischung über Nacht (15 h) bei 25°C gerührt.

Anschließend wurde die Mischung bei 25°C nacheinander zunächst mit 91,3 g (0,25 mol) 10 %-iger Salzsäure und anschließend mit 29 g (0,5 mol) Propionaldehyd versetzt. Nach 4 h Rühren bei 60°C wurde die Reaktionsmischung wie bei Beispiel 1 angegeben aufgearbeitet. Man erhielt so 145 g Rohprodukt, welches nach GC-Analyse 31,0 % 9-Acetoxynonenal und 7,7 % 9-Hydroxynonenal enthielt (Ausbeute an Wertprodukt insgesamt 60 %).

### Beispiel 6

### Herstellung von 9-Acetoxynonenal und 9-Hydroxynonenal

O=CH-CH=CH-(CH₂)₅-CH₂-OR (R = H, H₃CCO)

Analog zu den in Beispiel 5 beschriebenen Bedingungen aber in Abwesenheit von Propionaldehyd erhielt man bei der Verwendung von 219 g (0,6 mol) 10 %-iger Salzsäure und in Anwesenheit von 145 g (2,5 mol) Aceton 86 g Rohprodukt, welches nach GG-Analyse 8,8 % 9-Acetoxynonenal und 13,5 % 9-Hydroxynonenal enthielt (Ausbeute an Wertprodukt insgesamt 23 %). Außerdem enthielt dieses Rohprodukt 12,1 % an unumgesetztem 9-Hydroxynonenal-neopentylacetal.

### Beispiele 7 bis 10

### Herstellung von 9-Acetoxynonenal

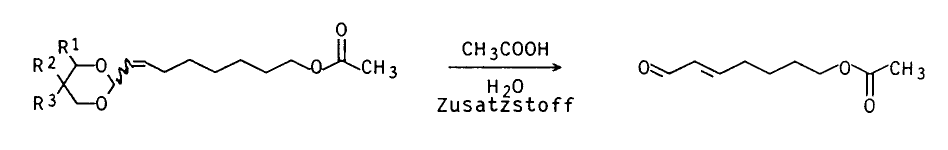

zu 72 g Essigsäure in 100 g Wasser werden 0,25 mol Acetal und 0,3 mol Zusatzstoff zugegeben. Man erwärmt 1 h zum Rückfluß, kühlt ab, gibt 500 ml Toluol zu, trennt die wäßrige Phase ab und neutralisiert durch Waschen mit gesättigter NaHCO₃-Lösung. Nach dem Einengen verbleiben Rohprodukte folgender Zusammensetzung:

| Zusatzstoff | R¹ | R² | R³ | Acetal | Aldehyd |
|---|---|---|---|---|---|
| - | H | CH₃ | CH₃ | 12,6 % | 87,4 % |
| Aceton | H | CH₃ | CH₃ | 8,8 % | 91,2 % |
| Propionaldehyd | H | CH₃ | CH₃ | 4,0 % | 96,0 % |
| Propionaldehyd | CH₃ | H | H | 4,2 % | 95,8 % |

### Beispiel 11

### Herstellung von Retinal

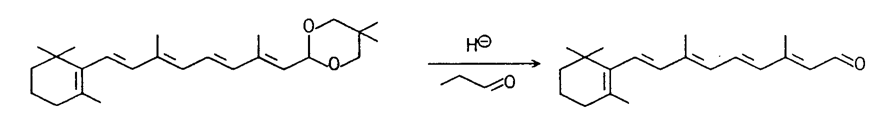

Eine Lösung von 0,25 Mol Retinalneopentylglykolacetal in 300 ml Heptan werden unter Rühren mit 100 ml 2 %iger Schwefelsäure, 150 ml Isopropanol und 17,4 g (0,3 Mol) Propanal versetzt und 2 Stunden bei 65°C gerührt. Nach HPLC liegt der Acetalgehalt bei < 1 %. Das gebildete Retinal liegt in einer Mischung aus mehreren Stereoisomeren vor. Das Verhältnis der Stereoisomeren (alltrans, 9 cis, 11 cis, 13 cis und di-cis) ist abhängig vom Ausgangsmaterial, sowie von der Dauer der Hydrolyse. Säure und höhere Temperatur isomerisieren letzlich bis zum Gleichgewicht der Stereoisomeren. Zur Aufarbeitung werden dem Ansatz 250 ml Wasser zugesetzt, um die Löslichkeit von Retinal in der Isopropanol/Wasserphase zu erniedrigen. Nach Abtrennen der Unterphase erhält man das in Heptan gelöste Retinal in 97 % Ausbeute.

## Patentansprüche

1. Verfahren zur Herstellung α,β-ungesättigter Aldehyde und Ketone der allgemeinen Formel I,
O=C(R¹)-CR²=CR³R⁴ I
in der die Substituenten R¹, R2, R³ und R⁴ unabhängig voneinander für Wasserstoff, ggf. subst. Alkyl- oder ggf. subst. Arylgruppen stehen, durch saure Hydrolyse von entsprechenden cyclischen α,β-ungesättigten Acetalen der allgemeinen Formel II, 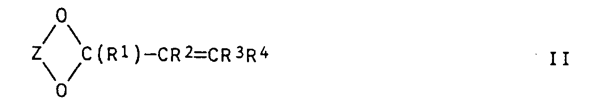 in der Z für eine ggf. subst. C₂- oder C₃-Kohlenstoffkette steht, dadurch gekennzeichnet, daß man die Hydrolyse in Gegenwart von gesättigten Aldehyden durchführt.

2. Verfahren nach Anspruch 1, dadurch gekennzeichnet, daß Z für eine 1,3-(2,2-Dimethyl)-propylenkette steht.

3. Verfahren nach Anspruch 1, dadurch gekennzeichnet, daß man die gesättigten Aldehyde in einer Menge von 1 bis 3 mol-Äq. bezogen auf das eingesetzte cyclische α,β-ungesättigte Acetal bzw. Ketal der allgemeinen Formel II verwendet.

4. Verfahren nach Anspruch 1, dadurch gekennzeichnet, daß man als gesättigte Aldehyde unverzweigte oder verzweigte C₁-C₆-Aldehyde verwendet.

5. Verfahren nach Anspruch 1, dadurch gekennzeichnet, daß man die Hydrolyse bei Temperaturen von 0 bis 150°C durchführt.

6. Verfahren nach Anspruch 1, dadurch gekennzeichnet, daß man die Hydrolyse in inerten organischen Lösungsmitteln durchführt.

7. Verfahren nach Anspruch 5, dadurch gekennzeichnet, daß man die Hydrolyse in aprotischen Lösungsmitteln durchführt.

8. Verfahren nach Anspruch 1, dadurch gekennzeichnet, daß man die Hydrolyse mit C₁₋₆ organischen Säuren oder mit Mineralsäuren durchführt.

9. Verfahren nach Anspruch 1, dadurch gekennzeichnet, daß man die Hydrolyse mit katalytischen Mengen C₁₋₆ organischer Säuren oder Mineralsäuren durchführt.

## Claims

1. A process for the preparation of an α,β-unsaturated aldehyde or ketone of the general formula I
O=C(R¹)-CR²=CR³R⁴ I
in which the substituents R¹, R², R³, and R⁴ independently of one another are hydrogen, unsubstituted or substituted alkyl, or optionally substituted aryl, by acid hydrolysis of the corresponding cyclic α,β-unsaturated acetal of the general formula II 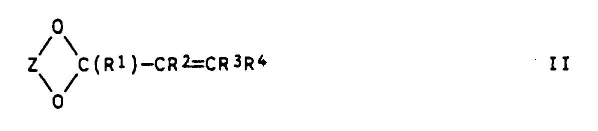 in which Z is an unsubstituted or substituted C₂ or C₃ carbon chain, wherein the hydrolysis is carried out in the presence of a saturated aldehyde.

2. A process as claimed in claim 1, wherein Z stands for a 1,3-(2,2-dimethyl)propylene chain.

3. A process as claimed in claim 1, wherein the saturated aldehyde is used in an amount of from 1 to 3 molar equivalents, based on the cyclic α,β-unsaturated acetal or ketal of the general formula II used.

4. A process as claimed in claim 1, wherein the saturated aldehyde used is an unbranched or branched C₁-C₆-aldehyde.

5. A process as claimed in claim 1, wherein the hydrolysis is carried out at a temperature of from 0 to 150°C.

6. A process as claimed in claim 1, wherein the hydrolysis is carried out in an inert organic solvent.

7. A process as claimed in claim 5, wherein the hydrolysis is carried out in an aprotic solvent.

8. A process as claimed in claim 1, wherein the hydrolysis is carried out using a C₁-C₆-organic acid or a mineral acid.

9. A process as claimed in claim 1, wherein the hydrolysis is carried out using a catalytic amount of a C₁-C₆-organic acid or a mineral acid.

## Revendications

1. Procédé de préparation d'aldéhydes et de cétones insaturés en α,β, de la formule générale I
O=C(R¹)-CR²=CR³R⁴ (I)
dans laquelle les substituants R¹, R², R³ et R⁴ sont mis, indépendamment l'un de l'autre, pour hydrogène, groupes alkyle ou aryle éventuellement substitués, par hydrolyse acide d'acétales cycliques, insaturés en α,β, correspondants, de la formule générale II 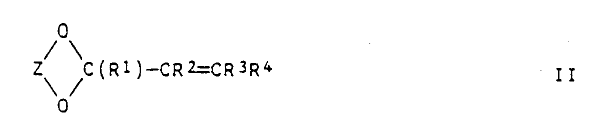 dans laquelle Z est mis pour une chaîne de carbone en C2 ou C3, éventuellement substituée, caractérisé par le fait que l'on effectue l'hydrolyse en présence d'aldéhydes saturés.

2. Procédé selon la revendication 1, caractérisé par le fait que Z est mis pour une chaîne 1,3-(2,2-diméthyl)-propylène.

3. Procédé selon la revendication 1, caractérisé par le fait que l'on utilise les aldéhydes saturés en quantité de 1 à 3 équimolaire, rapportés à l'acétale ou cétale insaturé en α,β, cyclique, utilisé, de la formule générale II.

4. Procédé selon la revendication 1, caractérisé par le fait que l'on utilise, comme aldéhydes saturés, des aldéhydes en C1-C6 ramifiés ou non ramifiés.

5. Procédé selon la revendication 1, caractérisé par le fait que l'on effectue l'hydrolyse à des températures de 0 à 150°C.

6. Procédé selon la revendication 1, caractérisé par le fait que l'on effectue l'hydrolyse dans des solvants organiques inertes.

7. Procédé selon la revendication 5, caractérisé par le fait que l'on effectue l'hydrolyse dans les solvants aprotiques.

8. Procédé selon la revendication 1, caractérisé par le fait que l'on effectue l'hydrolyse avec des acides organiques en C1-C6 ou des acides minéraux.

9. Procédé selon la revendication 1, caractérisé par le fait que l'on effectue l'hydrolyse avec des quantités catalytiques d'acides organiques en C1-C6 ou d'acides minéraux.
